# EUROPEAN PATENT APPLICATION

(11) **EP 1 762 192 A2**
(43) Date of publication of application: **14.03.2007**
(21) Application number: 06445063.8
(22) Date of filing: 12.09.2006
(51) Int. Cl.: A61B 17/16

(54) **Surgical abrader with clear hood**

(30) Priority: 12.09.2005 US 715615 P
(71) Applicant: Arthrex, Inc., Naples, Florida 34108-1945 (US)
(72) Inventor: Benavitz, William C., Naples, Florida 34119 (US); Adams, Kenneth M., Naples, Florida 34120 (US); O'Quinn, Philip S., Naples, Florida 34116 (US)
(74) Representative: Janson, Ronny

(57) **Abstract**

A rotary abrader allowing for improved visibility during surgery and improved aspiration of waste material. This is accomplished by providing a hood or
sheath (79) formed of a clear material, which is available in various shapes and sizes. The abrader comprises an outer tube (52) and an inner tube (102). Slots (73,74) extending from the lumen (53) to the outer surface (55) of the outer tube permit aspiration of waste material and are used to attach the hood to the outer tube. The inner and outer tubes form part of inner and outer assemblies which each have a hub (122,120) formed at their proximal end. An abrading element (116 ) is located at the distal end of the inner tube, which is rotatable.

## Description

### FIELD OF THE INVENTION

The present invention relates to rotary abraders used in surgery and, more particularly to an abrader which gives the surgeon an improved view of the surgical site during arthroscopic procedures.

### BACKGROUND OF THE INVENTION

Least invasive surgical techniques have gained significant popularity because of their ability to accomplish outcomes with reduced patient pain and accelerated return of the patient to normal activities. Arthroscopic surgery, in which the intra-articular space is filled with fluid, allows orthopedists to efficiently perform procedures using special purpose instruments designed specifically for arthroscopists. Among these special purpose tools are various manual graspers and biters, electrosurgical devices, and powered shaver blades and rotary abraders. Shaver blades having hollow bores are typically removably coupled to a shaver handpiece and are used for cutting, resecting, boring, and abrading both soft and hard tissue at the surgical site. An arthroscopic abrader (also known as a burr) generally includes a rotatable inner tube having an abrading head at its distal end and fixed outer tube for rotatably receiving the inner tube. Abraders are used for abrading or shaping both soft and hard tissue as bone, cartilage, ligaments, etc. by use of the rotating abrading head. As the tissue is being abraded, debris and fluid are generally drawn or sucked through the rotatable inner shaft which supports the burr.

Requirements for a rotary abrader for arthroscopy include a compact size so as to fit through small cannulae, a means for removal of debris, and a configuration which allows the surgeon to access, while retaining good visibility, structures within a joint. One requirement for good visibility is the effective removal of debris as it is generated. Another is that the instrument be configured so that the view of the active portion of the abrader in contact with the tissue and the view of the tissue being abraded are not obscured by the instrument.

Rotary abraders for arthroscopy generally have a shield, also called a "hood," on one side of the distal end of the outer tube to prevent inadvertent damage to tissue in close proximity to the tissue being abraded. The distal end of this hood is angled with respect to the tube axis so as to expose only one side of the burr head. During use, the burr head (the abrading element at the distal end of the rotating inner member) is subjected to significant lateral forces. Although rotary abraders typically have a bearing near the distal end of the instrument to support the inner member, lateral deflection of the burr head occurs to some degree. Contact between the burr head and the hood is undesirable since the burr will abrade metal from the hood and deposit metallic debris in the joint. Accordingly, it is necessary to leave adequate clearance between the hood and the burr head. Further, hoods are usually opaque, which hinder visibility of the surgical site during surgery.

Removal of debris from the field is accomplished by aspirating the material from the joint via a lumen in the inner, rotating member which is connected through a means in the handpiece to an external vacuum source. The aspiration of material through the inner member is desirable as this allows easy transfer of the materials from the proximal end of the instrument to the aspiration passage of the handpiece. The manner in which material and fluid enter the lumen at the distal end of the instrument has a large effect on the volume of flow through the instrument and on the frequency with which the instrument clogs. Insufficient flow causes decreased visibility because of residual debris suspended in the intra-articular fluid. Clogging requires that the instrument be removed from the joint and "de-clogged." The degree of difficulty of clog removal is determined by the instrument design. Even if clog removal is easily accomplished, removing, de-clogging and reinserting the instrument is a nuisance and causes increased procedure times. Aspiration effectiveness, and therefore instrument design, have a large effect on burr efficiency.

There is a need for an improved rotary abrader that provides a clear hood to improve visibility during surgery.

It is accordingly an object of this invention to produce a rotary abrader with a hood or sheath or guard that is available in various shapes to improve the procedure of abrading tissue.

Further, there is a need for an abrading instrument having rigidity, and an aspiration means which effectively removes debris without clogging and which can be readily cleared of clogs without disassembly, and which enhances surgeon visibility in procedures where visibility is crucial, such as SLAP repair.

### SUMMARY OF THE INVENTION

The present invention is a rotary abrader having a clear sheath or hood over the abrading element or burr. Slots provided on the cannulated shaft supporting the burr provide aspiration and are used to attach the hood to the cannulated shaft.

Several hoods in various shapes are provided to cover the burr at the distal end of the abrader. The hoods are formed of a clear material, such as polycarbonate. The clear material of the hoods provide visibility of the burr during the procedures.

Other features and advantages of the present invention will become apparent from the following detailed description of the invention, which is provided with reference to the accompanying drawings.

### BRIEF DESCRIPTION OF THE DRAWINGS

FIG. 1 is a plan view of the outer tube of a rotary abrader in accordance with an embodiment of the invention.

FIG. 2 is a side elevational view of an outer tube of the rotary abrader of FIG. 1.

FIG. 3 is a side elevational view of the hood of an outer tube assembly of the rotary abrader of FIG. 1.

FIG. 4 is a plan view of the hood of an outer tube assembly of the rotary abrader of FIG. 1.

FIG. 5 is a perspective view of the hood of the outer tube assembly of the rotary abrader of FIG. 1.

FIG. 6 is a plan view of the outer tube of the rotary abrader of FIG. 1.

FIG. 7 is a plan view of the outer tube of the rotary abrader of FIG. 1.

FIG. 8 is a plan view of the outer tube of the rotary abrader of FIG. 1.

FIG. 9 is an expanded axial view of the objects at location A-A of FIG. 6.

FIG. 10 is a plan view of the inner tube assembly of the rotary abrader.

FIG. 11 is an expanded plan view of the distal end of the inner tube assembly of the rotary abrader.

FIG. 12 is a perspective view of the distal end of the inner tube assembly of the rotary abrader.

FIG. 13 is an expanded plan view of the distal end of the inner tube assembly of a rotary abrader in accordance with another embodiment of the invention.

FIG. 14 is an expanded plan view of the distal end of the inner tube assembly of a rotary abrader in accordance with another embodiment of the invention.

FIG. 15 is a perspective view of the distal end of the inner tube assembly of a rotary abrader in accordance with another embodiment of the invention.

FIG. 16 is a plan view of the inner tube of a rotary abrader.

FIG. 17 is a plan view of the assembled rotary abrader.

FIG. 18 is a side elevational view of an assembled rotary abrader.

FIG. 19 is a plan view of an outer tube assembly of a rotary abrader in accordance with another embodiment of the invention.

FIG. 20 is a side elevational view of an outer tube assembly of the rotary abrader of FIG. 19.

FIG. 21 is a plan view of an inner tube assembly of a rotary abrader.

FIG. 22 is a plan view of an outer tube assembly of the rotary abrader of FIG. 19.

FIG. 23 is a plan view of a hood of the rotary abrader of FIG. 19.

FIG. 24 is a side elevational view of the hood of a rotary abrader of FIG. 19.

FIG. 25 is a perspective view of a hood of the rotary abrader of FIG. 19.

### DETAILED DESCRIPTION OF THE INVENTION

In the following detailed description, reference is made to various specific embodiments in which the invention may be practiced. These embodiments are described with sufficient detail to enable those skilled in the art to practice the invention, and it is to be understood that other embodiments may be employed, and that structural and logical changes may be made without departing from the spirit or scope of the present invention.

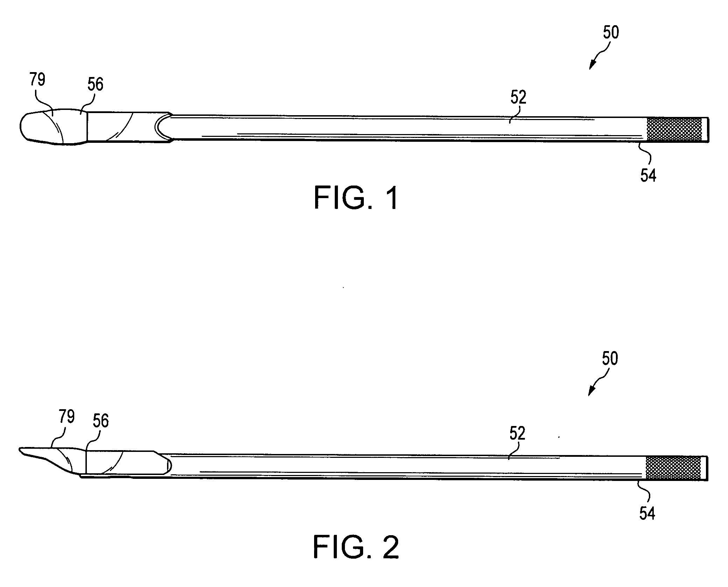

Referring now to the drawings, where like elements are designated by like reference numerals, FIGS. 1 through 5 illustrate the outer tubular portion 50 of a rotary abrader formed in accordance with the first embodiment of the invention. Outer tube 52 has a proximal end 54 and a distal end 56. Tube 52 has a lumen 53 of diameter 60 and an outer diameter 62. Distal end 56 has a first portion 64 with an inner diameter 66 formed therein, and a second portion 68 of length 70 with a diameter 72 formed therein. Diameter 72 decreases slightly and varies at slope 45 at distal end 56. Diameter 72 is slightly larger than diameter 60. Diameter 66 is slightly larger than diameter 72. Beveled surfaces 75 and 77 together with outer surfaces 55 define a hood (or guard) 79.

The hood 79 may be formed of any material, but in an exemplary embodiment, at least a portion of hood 79 is made of a clear polymer plastic material, such as polycarbonate. The polycarbonate hood 79 enhances visualization of the operational site. The hood 79 also is provided to enhance aspiration and to protect tissue surrounding the operational site.

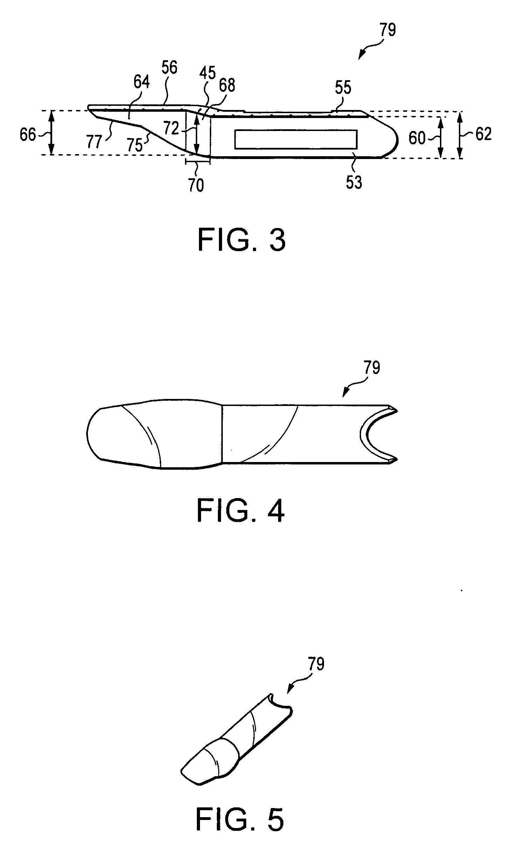

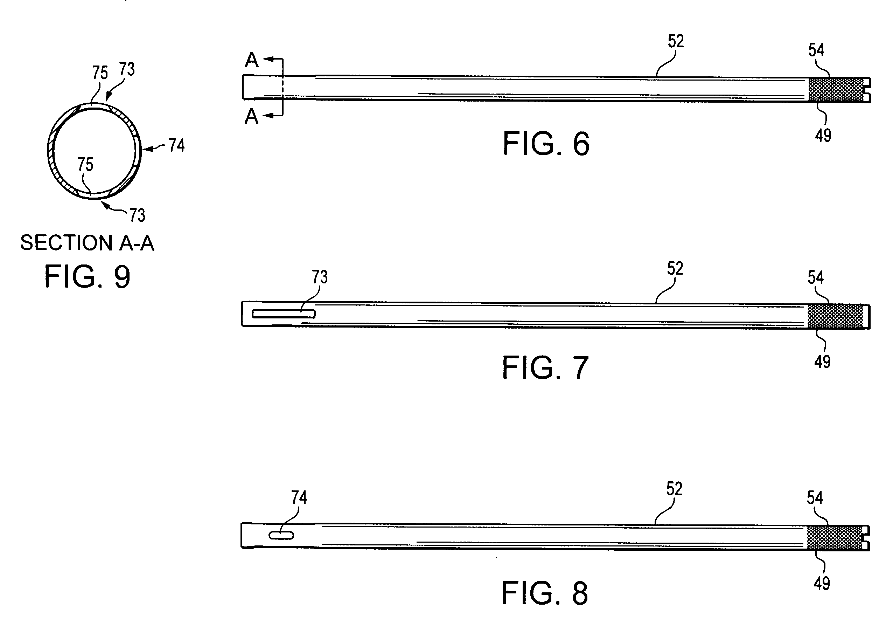

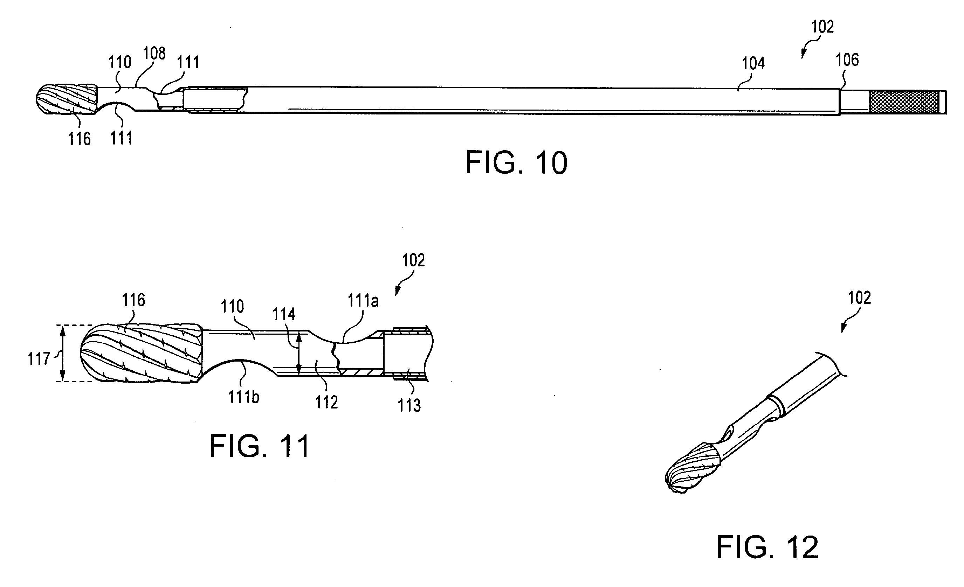

The outer tube 52, as illustrated in FIGS. 6 through 9, is preferably about 5.15 inches long and has a diameter of about 0.171 inches. Further, elongated slots 73 and 74 extend from the lumen 53 to the tube outer surface 55 (FIG. 3). Two elongated slots 73 are used to attach hood 79 to the outer tube 52. As shown in FIG. 9, slots 73 have inward bevels for insert molding, created by laser cutting, to allow the plastic from hood 79 to fill the space 75. Also, the slots 73, having a dovetail configuration, prevent the hood 79 from becoming dislodged. A core pin is inserted into the lumen of the outer tube 52 to prevent plastic from obstructing the pathway for inner tube 102 (FIG. 10). Slot 74 provides aspiration of debris and waste without clogging. A raised diamond knurl 49 at the proximal end 54 of the outer tube 52 is a point of attachment for the outer tube 52 with the inner tube 102 (FIG. 10).

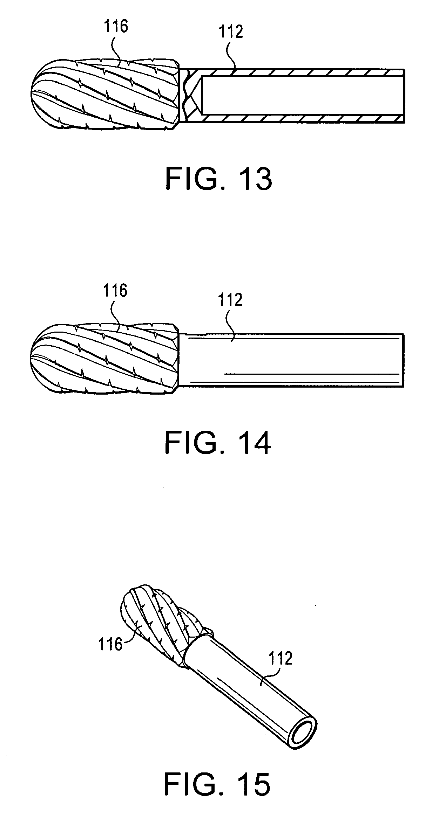

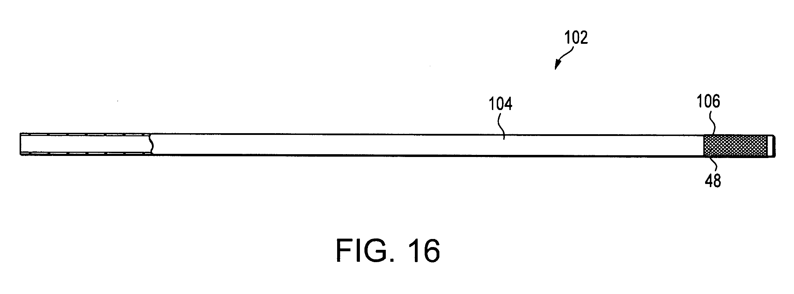

Referring now to FIGS. 10 through 12, inner tube assembly 102 of a rotary abrader constructed in accordance with the principles of the invention has an elongated tubular portion 104 with a proximal end 106 and a distal end 108. Distal end 108 has affixed thereto portion 110 having a diameter 114, and a distal portion 116 forming an abrading element (or burr head) of diameter 117. In addition, a Teflon (FEP) shrink tubing covers the elongated tubular portion of inner tube assembly 102 to prevent wear and keep the inner and outer tubes more concentric. Near distal end 108 of tubular portion 104 aspiration port 111a extends from lumen 112 to outer surface 113. Aspiration port 111b extends from the distal portion 116 to a distance immediately before 111a. The suction pathways 111a and 111b enhance aspiration of waste and debris. An example of a surgical abrader that provides a suction port proximal to the bearing to enhance aspiration is disclosed in U.S. Patent No. 7,077,845. In another preferred embodiment, as illustrated in FIGS. 13 through 15, the burr head and portion 112 extends about 1 inch without aspiration ports at the distal portion 116. The aspiration ports may be disposed on the inner tube a distance away from the burr head. In addition, as best seen in FIG. 16, the inner tube 102 has an elongated tubular portion 104 that extends about 5.4 inches. A raised diamond knurl 48 at the proximal end 106 of the inner tube 102 is a point of attachment for the inner tube 102 with the outer tube 52 (FIG. 1).

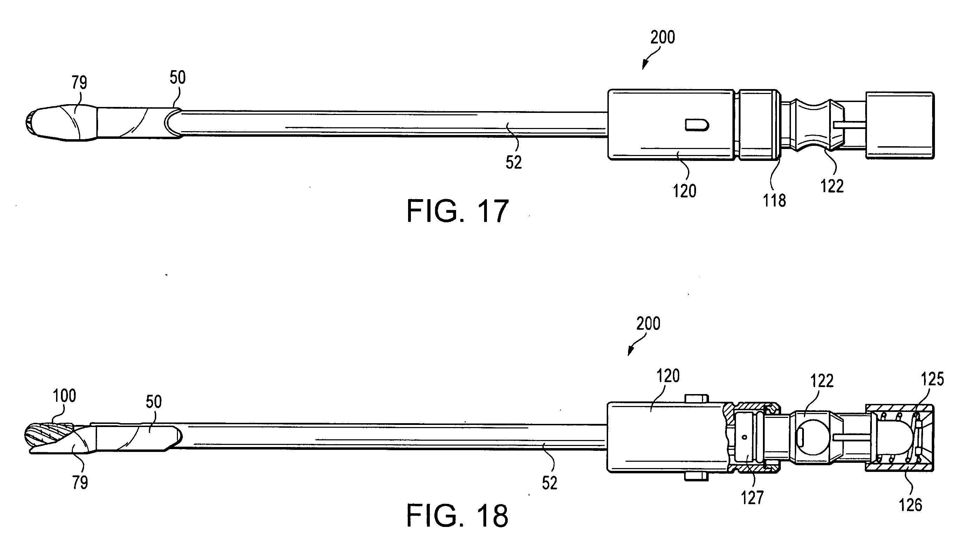

Referring now to FIGS. 17 and 18, an assembled rotary abrader 200 is shown. To assemble, an inner tube assembly 100 is inserted into the outer tube 52 of outer assembly 50. Inner hub 122 is inserted into the outer hub 120, which is held secure by a retaining ring 118. The inner hub 122 of inner tube assembly 100, includes a spring 125, spring retainer 126, and thrust washer 127. Also, the clear tip hood 79 covers the burr blade.

In another embodiment, the clear tip hood is removably interchangeable with other hoods, which are provided in various shapes. U.S. Patent Application No. 11/365,939, provides another embodiment of an endoscopic rotary abrader with an abrader and an outer assembly portion having flush ends and a removable hood.

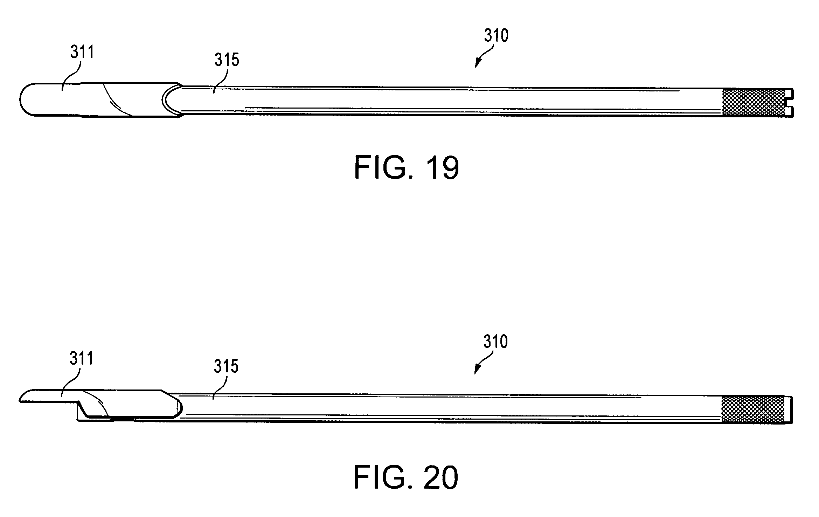

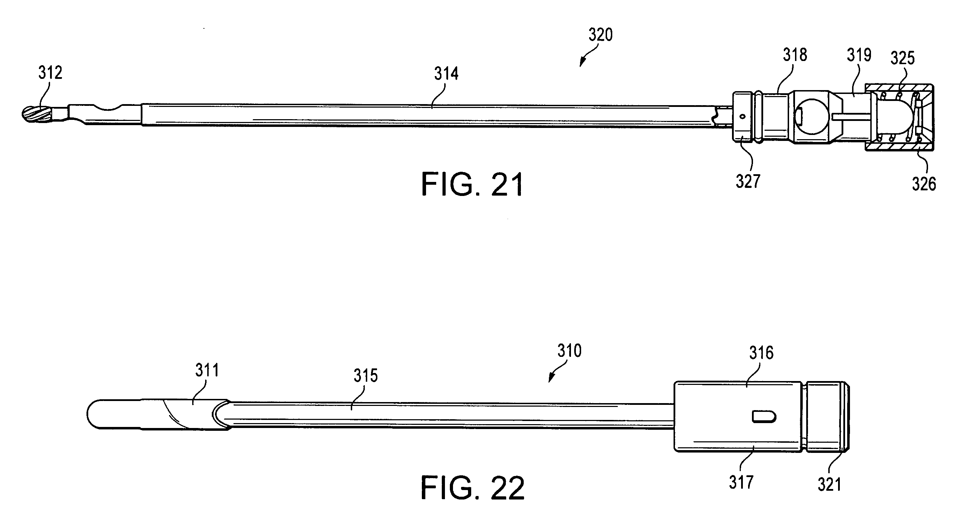

In another preferred embodiment, as illustrated in FIGS. 19 through 25, a rotary abrader has an outer assembly 310 and inner assembly 320. A hood 311 is insert molded onto the outer tube 315. The hood 311 covers the burr blade 312. This abrader is known as a SLAP burr. In this embodiment, the inner tube 314 is removable from the outer tube 315. The outer assembly 310 has a proximal end 316 with a hub 317 affixed to the proximal end 316 of outer tube 315. The inner assembly 320 has a proximal end 318 with a hub 319 affixed to the proximal end 318 of inner tube 314. As discussed above, to assemble the rotary abrader, the inner tube assembly 320 is inserted into the outer tube 315 of outer assembly 310. Inner hub 319 is inserted into the outer hub 317, which is held secure by a retaining ring 321. The inner hub 319 of inner tube assembly 320, includes a spring 325, spring retainer 326, and thrust washer 327.

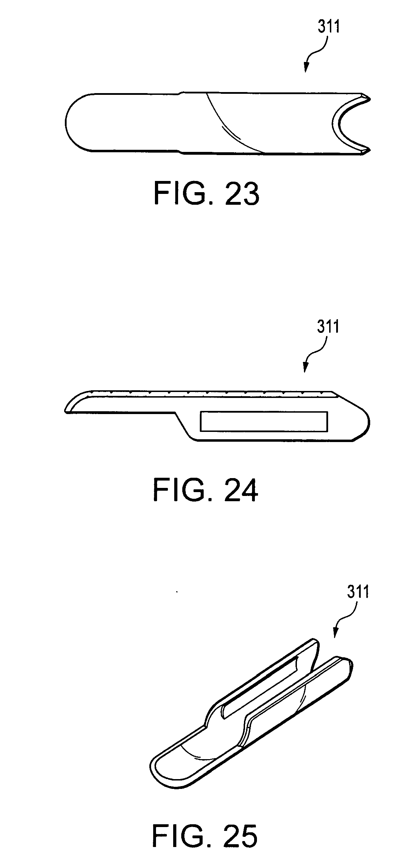

As best seen in FIGS. 23 through 25, the hood 311 has a different shape than the embodiment discussed above. This shape provides better protection for the surrounding tissue as the burr blade abrades the tissue. The hood is preferably made of a polycarbonate or other clear material to enhance visibility of the surgical site. Although the hood is shaped differently than the embodiments described above, the abrader still maintains the required minimum clearance between the burr head and the hood and does not obstruct the surgeon's view. In a preferred embodiment, the hood is enlarged as compared to the diameter of the outer tube. In this embodiment, the diameter of the abrading element can be increased and still maintain the minimum clearance required between the element and the hood.

Although the present invention has been described in relation to particular embodiments thereof, many other variations and modifications and other uses will become apparent to those skilled in the art. Therefore, the present invention is to be limited not by the specific disclosure herein, but only by the appended claims.

## Claims

1. An apparatus for abrading tissue, comprising:
an inner assembly rotatably positioned within an outer assembly;
a hub formed at a proximal end of each of the inner assembly and the outer assembly;
an abrading element located at a distal end of an inner tube of the inner assembly; and
a hood located at a distal end of an outer tube of the outer assembly, wherein the inner tube and the outer tube rotate concentrically and wherein the hood is formed of a clear material.

2. The apparatus of claim 1, wherein the hood is comprised of polycarbonate.

3. The apparatus of claim 1, wherein the hood is comprised of an outer surface of the outer tube and two beveled edges on the opposite side of the outer tube from the outer surface composing the hood.

4. The apparatus of claim 1, further comprising at least one aspiration port, located near the distal end of the inner tube and extending axially from a lumen of the inner tube to an outer surface of the inner tube.

5. The apparatus of claim 4, further comprising at least one slot in the outer tube of the outer assembly, the at least one slot extending radially from a lumen of the outer tube to an outer surface of the outer tube.

6. The apparatus of claim 5, wherein debris may be aspirated through the at least one slot into a lumen of an inner tube via suction.

7. The apparatus of claim 5, wherein the at least one slot may be used to attach a hood to the outer tube.

8. The apparatus of claim 4, wherein debris may be aspirated through the at least one aspiration port into the lumen of the inner tube via suction.

9. The apparatus of claim 1, wherein the inner tube is coated with Teflon shrink tubing.

10. The apparatus of claim 8, wherein the apparatus is made from a material which will not degrade during sterilization.

11. An apparatus for abrading tissue, comprising:
an inner assembly rotatably positioned within an outer assembly, wherein an inner tube of the inner assembly is removable from an outer tube of the outer assembly;
a hub formed at a proximal end of each of the inner assembly and the outer assembly;
an abrading element located at a distal end of an inner tube of the inner assembly; and
a hood located at a distal end of an outer tube of the outer assembly, wherein the hood is formed of a clear material.

12. The apparatus of claim 11, wherein the hood is comprised of polycarbonate.

13. The apparatus of claim 11, wherein the hood may be different sizes to accommodate the abrading element.

14. The apparatus of claim 11, wherein a radius of the hood is greater than a radius of the outer tube assembly.

15. The apparatus of claim 11, further comprising at least one aspiration port, located near the distal end of the inner tube and extending axially from a lumen of the inner tube to an outer surface of the inner tube.

16. The apparatus of claim 15, wherein debris may be aspirated through the at least one aspiration port into the lumen of the inner tube via suction.
